# FASCICULE DE BREVET EUROPEEN

(11) **EP 2 456 866 B1**
(45) Date de publication et mention de la délivrance du brevet: **19.10.2016**
(21) Numéro de dépôt: 10752057.9
(22) Date de dépôt: 20.07.2010
(51) Int. Cl.: C12N 9/24

(54) **PORPHYRANASES ET LEUR UTILISATION POUR HYDROLYSER DES POLYSACCHARIDES**
PORPHYRANASEN UND IHRE VERWENDUNG ZUR HYDROLYSE VON POLYSACCHARIDEN
PORPHYRANASES, AND USE THEREOF FOR HYDROLYZING POLYSACCHARIDES

(30) Priorité: 21.07.2009 FR 0955070
(43) Date de publication de la demande: 30.05.2012
(73) Titulaire: Centre National de la Recherche Scientifique (CNRS), 75016 Paris (FR)
(72) Inventeur: HEHEMANN, Jan-Hendrik, F-29682 Roscoff (FR); CORREC, Gaëlle, F-29250 Santec (FR); MICHEL, Gurvan, F-29680 Roscoff (FR); BARBEYRON, Tristan, F-29233 Cleder (FR); HELBERT, William, F-29680 Roscoff (FR); CZJZEK, Mirjam, F-29250 Plougoulm (FR)
(74) Mandataire: Lavoix
(86) Numéro de dépôt international: PCT/FR2010/051530
(87) Numéro de publication internationale: WO 2011/010062

(56) Documents cités:
- ZHONG Z ET AL: "Sequence analysis of a 101-kilobase plasmid required for agar degradation by a Microscilla isolate." APPLIED AND ENVIRONMENTAL MICROBIOLOGY DEC 2001 LNKD- PUBMED:11722934, vol. 67, no. 12, décembre 2001 (2001-12), pages 5771-5779, XP002577067 ISSN: 0099-2240
- DATABASE UniProt [Online] 1 décembre 2001 (2001-12-01), "SubName: Full=MS132, putative beta-agarase;" XP002577068 extrait de EBI accession no. UNIPROT:Q93P99 Database accession no. Q93P99
- HEHEMANN JH, CORREC G, BARBEYRON T, HELBERT W, CZJZEK M, MICHEL G: "Transfer of carbohydrate-active enzymes from marine bacteria to Japanese gut microbiota" NATURE, vol. 464, no. 7290, 8 avril 2010 (2010-04-08), pages 908-912, XP002577069 DOI: 10.1038
- ZHAO T ET AL: "Degradation of porphyran from Porphyra haitanensis and the antioxidant activities of the degraded porphyrans with different molecular weight" INTERNATIONAL JOURNAL OF BIOLOGICAL MACROMOLECULES, ELSEVIER BV, NL LNKD- DOI:10.1016/J.IJBIOMAC.2005.12.018, vol. 38, no. 1, 28 février 2006 (2006-02-28), pages 45-50, XP025096112 ISSN: 0141-8130 [extrait le 2006-02-28]
- HATADA YUJI ET AL: "Hyperproduction and application of alpha-agarase to enzymatic enhancement of antioxidant activity of porphyran" décembre 2006 (2006-12), JOURNAL OF AGRICULTURAL AND FOOD CHEMISTRY, VOL. 54, NR. 26, PAGE(S) 9895-9900 , XP002577070 ISSN: 0021-8561 Abstract; page 9895, colonne de gauche, alinéa 1 - page 9895, colonne de droite, alinéa 1
- AOKI YUKIHISA ET AL: "Preparation of recombinant polysaccharide-degrading enzymes from the marine bacterium, Pseudomonas sp ND137 for the production of protoplasts of Porphyra yezoensis", EUROPEAN JOURNAL OF PHYCOLOGY, CAMBRIDGE UNIVERSITY PRESS, CAMBRIDGE, DE, vol. 41, no. 3, 1 August 2006 (2006-08-01) , pages 321-328, XP002576421, ISSN: 0967-0262, DOI: 10.1080/09670260600801682
- ZHAO ET AL: "Extension of life span and improvement of vitality of Drosophila melanogaster by long-term supplementation with different molecular weight polysaccharides from Porphyra haitanensis", PHARMACOLOGICAL RESEARCH, ACADEMIC PRESS, LONDON, GB, vol. 57, no. 1, 23 December 2007 (2007-12-23), pages 67-72, XP022477360, ISSN: 1043-6618, DOI: 10.1016/J.PHRS.2007.12.001

## Description

La présente invention concerne l'isolation et la caractérisation de deux protéines ayant une nouvelle activité enzymatique, à savoir une activité porphyranase. Ces protéines sont utiles pour hydrolyser des polysaccharides contenant des agaro-colloïdes sulfatés et pour produire des oligo-porphyranes, notamment des oligo-porphyranes de structure et de taille définie.

Les polysaccharides tels que les agars (agarose, porphyrane, *etc.*) et les carraghénanes sont très largement utilisés en tant qu'agents gélifiants ou épaississants dans diverses branches d'activité, notamment dans l'industrie agro-alimentaire et cosmétique. Ainsi, environ 6000 tonnes d'agars et 22 000 tonnes de carraghénanes sont extraits annuellement des algues rouges marines à des fins agro-alimentaires. Les agars sont produits industriellement à partir des algues rouges des genres *Gelidium* et *Gracilaria.* Par ailleurs, l'algue rouge *Porphyra* (connue sous le nom populaire de nori) est l'algue la plus consommée dans le monde. La production annuelle est estimée à 90000 tonnes/an (poids sec), représentant un marché d'environ 1,5 milliard de dollars américains.

Les polysaccharides et leurs dérivés présentent également un intérêt dans le domaine thérapeutique. Ainsi, l'énoxaparine (Lovenox^{®}), lequel est commercialisé pour le traitement de troubles thromboemboliques, est un mélange de chaînes de polysaccharides sulfatés de longueurs variables et formées d'unités disacchariques récurrentes. Par ailleurs, certains polysaccharides et oligosaccharides sulfatés, tels que les oligo-fucanes sulfatés produits à partir de polysaccharides algaux, sont utiles en tant qu'agents anti-microbiens et/ou antiviraux chez l'homme (Ghosh et al. 2009 Glycobiology. 19:2-15).

Les agaro-colloïdes sont des polysaccharides complexes. Certaines espèces d'algues contiennent un agaro-colloïde donné comme composé majoritaire, comme par exemple l'algue *Porphyra,* qui contient comme constituant principal l'agaro-colloïde dénommé porphyrane. Par abus de langage, les polysaccharides extraits d'algues rouges sont parfois également dénommés porphyrane, alors qu'il s'agit en fait d'un mélange d'agarose et de porphyrane, le porphyrane étant le composé majoritaire.

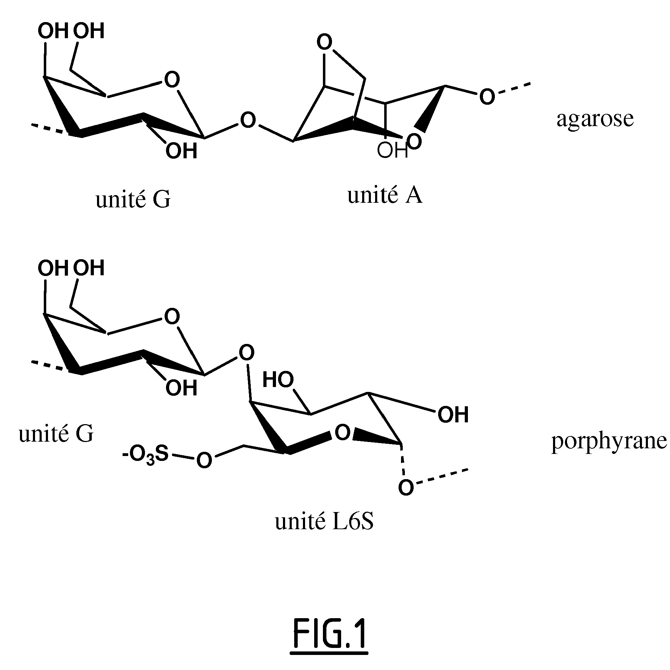

L'unité disaccharidique de base formant le porphyrane est constitué d'une unité D-galactose (unité G, voir Figure 1) lié en β-1,4 avec une unité L-galactose modifiée par une O-sulfatation sur la position 06 (unité L6S, voir Figure 1). Les unités disaccharides sont ensuite connectées entre elles par des liaisons α-1,3. Ce polysaccharide sulfaté est communément considéré comme le précurseur de l'agarose. En milieu naturel, d'autres modifications chimiques, comme la méthylation, donnent lieu à encore plus de variations de cette structure de base. Ces modifications chimiques ont pour conséquence de moduler le caractère gélifiant du polymère.

Jusqu'à présent, on connaît et utilise principalement des enzymes capables de dégrader les agars : les α-agarases et les β-agarases. Les β-agarases agissent sur la liaison β-1,4 (Jam et al. Biochem J. 2005 385 :703-13) et les α-agarases sur la liaison α-1,3 (Flament et al. Appl Environ Microbiol. 2007 73:4691-94) de l'unité disaccharidique agarose, et ces enzymes sont spécifiques des unités non substituées ou non modifiées. En effet, il existe de nombreuses bactéries, essentiellement marines, qui produisent des enzymes capables d'hydrolyser les agars (Michel et al., Appl Microbiol Biotechnol. 2006 71 :23-33). A partir de ces microorganismes, plusieurs gènes de β-agarases ont déjà été clonés et les protéines correspondantes surexprimées et purifiées.

Plus particulièrement, les premières β-agarases pleinement caractérisées biochimiquement et structuralement sont produites par une souche bactérienne isolée à partir de l'algue rouge *Delesseria sanguinea* (Jam et al. Biochem J. 2005 385 :703-13, Allouch et al. J Biol Chem. 2003 278:47171-80). Cette souche bactérienne a été déposée à la Collection DSMZ (Deutsche Sammlung von Mikroorganismen und Zellkulturen GmbH) le 8 mai 1998 sous le numéro DSM 12170. L'identification taxonomique de cette souche montre qu'elle définit un nouveau genre dans la classe des Flavobactéries et sa caractérisation lui a valu le nom de *Zobellia galactanivorans* (Barbeyron et al. Int J Syst Evol Microbiol. 2001 51:985-97). La κ-carraghénase de cette bactérie marine a également été clonée et caractérisée (Barbeyron et al. Mol Biol Evol. 1998 15:528-37).

En revanche, aucune enzyme spécifique des polysaccharides sulfatés tels que le porphyrane n'a encore été décrite à ce jour.

Par abus de langage, certaines enzymes capables de dégrader des polysaccharides extraits d'algues rouges contenant du porphyrane ont été incorrectement dénommées porphyranases dans la littérature scientifique. Cependant, ces enzymes possèdent en fait une activité agarase, et dégradent les agars présents dans les polysaccharides extraits d'algues rouges.

Ainsi, Lee et al. (2006 Annual Meeting and International Symposium of the Korean Society for Microbiology and Biotechnology) décrivent le clonage et le séquençage d'une enzyme de *Vibrio pelagius,* laquelle est dénommée porphyranase. Cependant, cette enzyme hydrolyse des liaisons β-1,4 D-galactosidiques, et conduit à l'obtention d'oligo-agaroses tels que le néoagarotetraose, le néoagarohexaose et le néoagarooctaose. Elle possède donc une activité agarase et non une activité porphyranase.

De façon similaire, Hatada et al. (2006 J. Agric. Food Chem. 54 :9895-9900) mentionnent les β-agarase et indiquent que celles-ci auraient une activité « porphyranase ». Cependant ces enzymes sont décrites comme capables d'hydrolyser les liaisons β-1,4 D-galactosidiques et de conduire à l'obtention d'oligomères d'agarose. Ces enzymes sont donc bien des agarases et non pas des porphyranases.

De même, Aoki et al. (2002 Marine and Highland Bioscience Center Report, 14:33-41) décrivent le clonage d'un gène codant pour une soi-disant porphyranase de *Pseudomonas sp.* ND 137. Cependant, le fait que la protéine correspondante dégrade le porphyrane n'a jamais été démontré, et sa forte identité de séquence (40%) avec l'agarase de *Pseudomonas sp.* CY24 prouve le contraire. De fait, la séquence de la protéine clonée par Aoki *et al.* est annotée en tant qu'agarase et non en tant que porphyranase dans l'entrée n° BAB79291.1 de la base de donnée de NCBI.

Certains produits de dégradation du porphyrane (oligo-porphyranes) ont pu être obtenus par hydrolyse chimique (Zhao et al. 2006 Int J Biol Macromol. 38:45-50). Ce procédé conduit toutefois à l'obtention d'un mélange très hétérogène d'oligo-porphyranes. De plus, l'hydrolyse est incomplète et le poids moléculaire moyen du mélange obtenu reste élevé.

Il existe donc un besoin en enzymes capables de catalyser l'hydrolyse de polysaccharides sulfatés tels que le porphyrane, et ce afin d'obtenir de nouveaux agents utilisables dans l'industrie agro-alimentaire, cosmétique et/ou pharmaceutique.

### DESCRIPTION DE L'INVENTION

Les inventeurs ont isolé et caractérisé les deux premières porphyranases décrites à ce jour. Bien qu'appartenant à la famille des GH16 des glycoside hydrolases, elles possèdent moins de 26% d'identité de séquence avec les autres membres de cette famille présents chez *Z. galactanivorans.*

Ces porphyranases, dénommées PorA et PorB, ont été clonées à partir de *Z*. *galactanivorans.* PorA et PorB sont représentées par les séquences SEQ ID NO : 2 et SEQ ID NO : 5 respectivement. Le domaine catalytique de PorA est 26.9 % identique à celui de PorB.

Il a été démontré que les protéines PorA et PorB possèdent une activité β-porphyranase, et qu'elles sont dénuées d'activité agarase. Une enzyme possédant une telle activité enzymatique n'avait jamais encore été décrite.

Il a également été démontré que les protéines PorA et PorB peuvent être utilisées pour hydrolyser du porphyrane et permettent l'obtention d'oligosaccharides de structure et de taille définies. Plus particulièrement, PorA et PorB permettent d'obtenir des mélanges homogènes de neo-porphyrobiose, neo-porphyrotetraose et/ou neoporphyrohexaose.

### Polypeptides selon l'invention

Les inventeurs ont isolé et caractérisé les premières porphyranases décrites à ce jour. De ce fait, la présente invention concerne un polypeptide isolé caractérisé en ce qu'il possède une activité porphyranase.

Dans le cadre de cette invention, on entend par « polypeptide » une molécule comprenant une chaîne linéaire d'acides aminés liés les uns aux autres par des liaisons peptidiques.

Par polypeptide (ou acide nucléique) « isolé », on entend ici un polypeptide (ou acide nucléique) isolé de l'organisme ou du microorganisme chez lequel ledit polypeptide (ou acide nucléique) est naturellement présent. Dans un mode de réalisation préféré, le polypeptide ou l'acide nucléique est sous forme isolée et purifiée. Dans un mode de réalisation particulièrement préféré, le polypeptide isolé est un polypeptide recombinant.

Par « porphyrane », on entend ici la molécule constituée d'un enchaînement d'unités disaccharidiques constitué d'une unité D-galactose lié en β-1,4 avec une unité L-galactose modifiée par une O-sulfatation sur la position 06.

Par « activité porphyranase », on entend ici la capacité à catalyser l'hydrolyse du porphyrane. Plus particulièrement, l'activité porphyranase des polypeptides selon l'invention consiste en une activité β-porphyranase, c'est-à-dire la capacité à catalyser le clivage des liaisons β-1,4 entre l'unité D-galactose (« unité G » sur la Figure 1) et l'unité L-galactose sulfatée en position 6 (« unité L6S » sur la Figure 1) de l'unité disaccharidique du porphyrane.

L'activité porphyranase peut par exemple être mesurée en réalisant une cinétique de la digestion du porphyrane. Une telle cinétique peut par exemple être réalisée comme décrit dans l'exemple 3. Plus particulièrement, une solution contenant 1 % (w/v) de polysaccharide préalablement digéré par une β-agarase peut être utilisée. 50 ml de la solution polysaccharidique peut par exemple être incubé à 30°C avec 6 µg d'enzyme. Des aliquotes peuvent être prélevés tout au long de l'hydrolyse jusqu'à ce que l'hydrolyse complète soit atteinte. La digestion du porphyrane par une porphyranase conduit à l'obtention d'oligo-porphyranes.

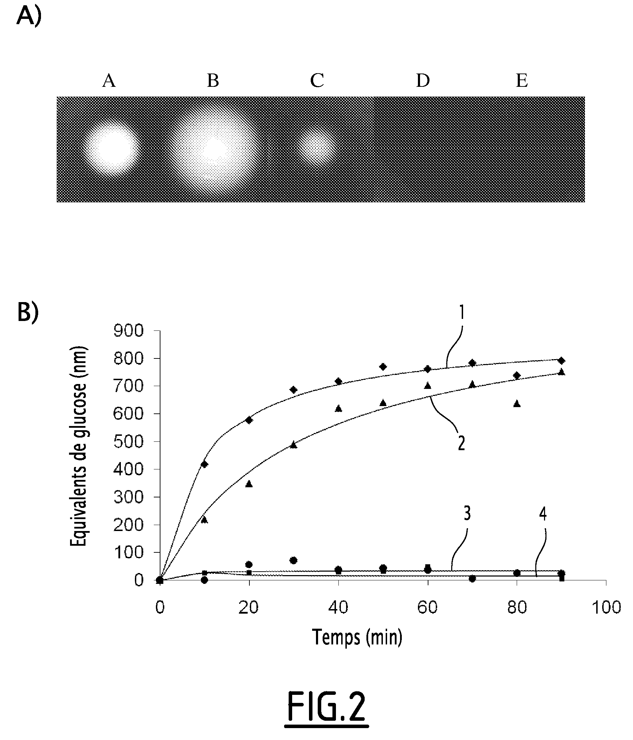

Les polypeptides selon l'invention sont préférentiellement dénués d'activité agarase. En d'autre terme, ces polypeptides catalysent spécifiquement l'hydrolyse de polysaccharides sulfatés. L'absence d'activité agarase peut facilement être mesurée, par exemple sur un gel comprenant 1% d'agarose (voir Figure 2). 1 µg d'enzyme peut par exemple être déposée sur un tel gel. Après une nuit d'incubation à 30°C, l'activité agarase peut être révélée par coloration au lugol.

Dans un mode de réalisation préféré, le polypeptide selon l'invention comprend ou consiste en une séquence choisie parmi :
- les résidus 19 à 274 de la séquence SEQ ID NO : 2 (lesquels correspondent à un fragment de PorA comprenant le domaine catalytique, fragment dénommé PorA_CM);
- les résidus 19 à 510 de la séquence SEQ ID NO : 2 (lesquels correspondent à la protéine PorA mature);
- les résidus 1 à 510 de la séquence SEQ ID NO : 2 (lesquels correspondent à la protéine PorA complète);
- les résidus 22 à 293 de la séquence SEQ ID NO : 5 (lesquels correspondent à la protéine PorB mature); ou
- les résidus 1 à 293 de la séquence SEQ ID NO : 5 (lesquels correspondent à la protéine PorB complète).

Les polypeptides incluent également des polypeptides dérivés des protéines PorA et PorB de séquence SEQ ID NO : 2 ou 5, étant entendu que ces polypeptides dérivés conservent l'activité porphyranase. De tels polypeptides dérivés comprennent ou consistent en une séquence choisie parmi :
a) une séquence au moins 26, 30, 40, 50, 60, 70, 80, 90, 95, 96, 97, 98 ou 99 % identique aux résidus :
   - 19 à 274 de la séquence SEQ ID NO : 2 ;
   - 19 à 510 de la séquence SEQ ID NO : 2 ;
   - 1 à 510 de la séquence SEQ ID NO : 2 ;
   - 22 à 293 de la séquence SEQ ID NO : 5 ; ou
   - 1 à 293 de la séquence SEQ ID NO : 5.
b) un fragment d'au moins 20, 50, 100, 150, 200, 210, 220, 230, 240 ou 250 acides aminés consécutifs de la séquence (a) ; et
c) un fragment d'au moins 20, 50, 100, 150, 200 210, 220, 230, 240 ou 250 acides aminés consécutifs d'une séquence consistant en les résidus:
   - 19 à 274 de la séquence SEQ ID NO : 2 ;
   - 19 à 510 de la séquence SEQ ID NO : 2 ;
   - 1 à 510 de la séquence SEQ ID NO : 2 ;
   - 22 à 293 de la séquence SEQ ID NO : 5 ; ou
   - 1 à 293 de la séquence SEQ ID NO : 5.

Par « séquence au moins 95% (par exemple) identique à une séquence de référence », on entend une séquence identique à la séquence de référence si ce n'est que cette séquence peut comporter jusqu'à cinq mutations ponctuelles (substitutions, délétions et/ou insertions) pour chaque partie de cent acides aminés de la séquence de référence. Ainsi, pour une séquence de référence faisant 100 acides aminés, un fragment de 95 acides aminés et une séquence de 100 acides aminés comportant 5 substitutions par rapport à la séquence de référence sont deux exemples de séquences 95% identiques à la séquence de référence.

Le pourcentage d'identité est généralement déterminé en utilisant un logiciel d'analyse de séquences. Le programme « needle », qui fait appel à l'algorithme d'alignement global « Needleman-Wunsch » pour trouver l'alignement optimal (avec gaps) de deux séquences sur la totalité de leur longueur, peut par exemple être utilisé. Ce programme est notamment disponible sur le site ebi.ac.uk.

Les polypeptides dérivés peuvent différer de la séquence de référence (en l'occurrence la séquence SEQ ID NO : 2 ou 4 ou l'un de ses fragments) par la présence de mutations de type délétion, insertion et/ou substitution d'acides aminés. Les substitutions peuvent être conservatives ou non conservatives.

Dans un mode de réalisation particulier, les polypeptides dérivés différent de la séquence de référence uniquement par la présence de substitutions conservatives. Les substitutions conservatives sont des substitutions d'acides aminés de même classe, telles que des substitutions d'acides aminés aux chaînes latérales non chargées (tels que l'asparagine, la glutamine, la serine, la cystéine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique), d'acides aminés aux chaînes latérales apolaires (tels que l'alanine, la valine, la leucine, l'isoleucine, la proline, la phénylalanine, la méthionine et le tryptophane).

Les polypeptides dérivés peuvent également correspondre à des variants allèliques des protéines PorA ou PorB de séquence SEQ ID NO : 2 ou 4, à des protéines homologues à PorA et PorB chez d'autres espèces que *Zobellia galactanivorans,* ou à des fragments de tels variants allèliques ou protéines homologues conservant l'activité porphyranase.

De manière optionnelle, les polypeptides comprennent un peptide signal. Si un peptide signal est présent, il peut être le peptide signal natif de la protéine (i.e. les résidus 1 à 18 de SEQ ID NO : 2 pour PorA, et les résidus 1 à 21 de SEQ ID NO : 5 pour PorB). Alternativement, le peptide signal peut être un peptide signal hétérologue à PorA ou PorB, par exemple un peptide signal convenant à l'expression de la protéine PorA ou PorB mature dans une cellule hôte donnée. Les séquences SEQ ID Nos : 2, 3, 5 et 6 sont des exemples de polypeptides selon l'invention comprenant soit un peptide signal natif (SEQ ID Nos : 2 et 5), soit un peptide signal hétérologue (SEQ ID Nos : 3 et 6).

Les polypeptides selon l'invention peuvent être préparés par purification à partir de leur organisme ou microorganisme d'origine, par synthèse chimique ou par génie génétique, et ce en utilisant les techniques bien connues de l'homme du métier. Dans un mode de réalisation préféré, les polypeptides selon l'invention sont obtenus par génie génétique.

### Acides nucléiques selon l'invention

La présente invention concerne également un acide nucléique isolé codant pour un polypeptide selon l'invention. Ce polypeptide peut correspondre à n'importe lequel des porphyranases décrites dans le paragraphe précédent.

Le terme « acide nucléique » se réfère aussi bien à des molécules d'ADN qu'à des molécules d'ARN, et incluse notamment des molécules d'ADNc et des molécules d'ARNm. L'acide nucléique peut être sous forme double brin (par exemple dans le cas d'un acide nucléique compris dans un vecteur d'expression) ou sous forme simple brin (par exemple dans le cas de sondes ou d'amorces).

Plus particulièrement, l'acide nucléique peut comprendre ou consister en une séquence choisie parmi :
a) les nucléotides 1 à 1533 ou 55 à 1533 de la séquence SEQ ID NO : 1 ;
b) les nucléotides 1 à 882 ou 64 à 882 de la séquence SEQ ID NO : 4 ; et
c) une séquence complémentaire à la séquence (a) ou (b).

L'acide nucléique peut également comprendre ou consister en des séquences dérivées des séquences SEQ ID NO : 1 ou 4. Les acides nucléiques dérivés incluent les acides nucléiques dont les séquences comprennent ou consistent en une séquence choisie parmi :
a) une séquence au moins 30, 40, 50, 60, 70, 80, 90, 95, 96, 97, 98 ou 99 % identique aux nucléotides :
   - 1 à 1533 ou 55 à 1533 de la séquence SEQ ID NO : 1 ; ou
   - 1 à 882 ou 64 à 882 de la séquence SEQ ID NO : 4.
b) un fragment d'au moins 60, 150, 300, 450, 600 ou 750 nucléotides consécutifs de la séquence (a) ;
c) un fragment d'au moins 60, 150, 300, 450, 600 ou 750 nucléotides consécutifs d'une séquence consistant en les nucléotides :
   - 1 à 1533 ou 55 à 1533 de la séquence SEQ ID NO : 1 ; ou
   - 1 à 882 ou 64 à 882 de la séquence SEQ ID NO : 4.
d) une séquence codée par un acide nucléique qui s'hybride à la séquence SEQ ID NO : 1 ou 4 dans des conditions de forte stringence ; et
e) une séquence complémentaire à l'une des séquences (a) à (d).

Le pourcentage d'identité entre deux séquences nucléotidiques est déterminé de la même manière que le pourcentage d'identité entre deux séquences d'acides aminés.

Les « conditions de forte de stringence » sont des conditions connues de l'homme du métier, et peuvent par exemple correspondre des conditions d'hybridations sur ADN lié à un filtre dans un tampon salin citrate de sodium (SSC) 5X, dodécyl sulfate de sodium (SDS) 2%, 100 microgrammes/mL d'ADN simple-brin, à 55-65°C pendant 8 heures, et lavage dans SSC 0,2X et SDS 0,2% à 60-65°C pendant 30 minutes.

Les séquences des acides nucléiques dérivés peuvent inclure des mutations telles que des substitutions, délétions et/ou insertions de nucléotides. Les substitutions peuvent soit être silencieuses, soit conduire à des mutations au niveau de la protéine codée par l'acide nucléiques. De préférence, les substitutions, délétions et/ou insertions au niveau de la séquence nucléotidique ne conduisent pas à un changement de phase de lecture, ni à l'introduction d'un codon-stop.

Dans un mode de réalisation préféré, l'acide nucléique dérivé est un acide nucléique codant pour la protéine PorA mature ou complète de séquence SEQ ID NO : 2, pour la protéine PorB mature ou complète de séquence SEQ ID NO : 5, ou pour des fragments de celles-ci conservant l'activité porphyranase, mais dont la séquence nucléotidique diffère de la séquence SEQ ID NO : 1 ou 4 en raison de la dégénérescence du code génétique et/ou en raison d'une variation allèlique.

Un autre mode de réalisation particulier préféré porte sur des acides nucléiques codant pour des protéines homologues de PorA ou PorB chez d'autres espèces que *Zobellia galactanivorans,* ou des fragments de celles-ci conservant l'activité porphyranase.

Un autre aspect porte sur des sondes et des amorces nucléotidiques comprenant ou consistant en un fragment de SEQ ID NO : 1 ou SEQ ID NO : 4. De telles sondes et amorces peuvent par exemple comprendre ou consister en 15 à 50 nucléotides consécutifs, préférentiellement 18 à 35 nucléotides consécutifs, de SEQ ID NO : 1 ou SEQ ID NO : 4. De telles sondes et amorces ne codent pas pour un polypeptide selon l'invention mais sont utiles pour le clonage, le séquençage et/ou la détection des acides nucléiques selon l'invention. Les sondes peuvent éventuellement être marquées, par exemple grâce à un marqueur radioactif ou à un fluorophore. Par ailleurs, les sondes et amorces peuvent comprendre, outre un fragment de la séquence SEQ ID NO : 1 ou SEQ ID NO : 4, une séquence hétérologue telle que la séquence d'un site de restriction ou une séquence de liaison à un marqueur.

Les acides nucléiques selon l'invention peuvent être préparés par synthèse chimique ou par génie génétique en utilisant les techniques bien connues de l'homme du métier et décrites, entre autres, dans Sambrook et al. ("Molecular Cloning: a Laboratory Manual" Ed. Cold Spring Harbor Press, N.Y., 1989). Les acides nucléiques selon l'invention peuvent par exemple être obtenus par amplification des gènes de *Zobellia galactanivorans* à l'aide de la méthode PCR (Polymerase Chain Reaction), comme décrit dans l'exemple 1. Le fragment d'acides nucléiques ainsi amplifié peut ensuite être cloné dans un vecteur d'expression selon les techniques décrites dans Maniatis. (« Molecular Cloning. A Laboratory Manual » New York, 1982) et/ou dans l'exemple 1.

### Vecteurs d'expression et cellules hôtes selon l'invention

L'invention concerne également des vecteurs d'expression comprenant un acide nucléique selon l'invention. Ces vecteurs d'expression, qui peuvent par exemple être des plasmides, comportent, outre la séquence d'acide nucléique selon l'invention, les moyens nécessaires à son expression. Ces moyens peuvent par exemple inclure un promoteur et un terminateur de transcription. Le vecteur d'expression peut également comporter d'autres éléments tels qu'une origine de réplication, un site de clonage multiple, un enhanceur, un peptide signal qui pourra être fusionné en phase avec le polypeptide de l'invention lors du clonage, et un ou plusieurs marqueurs de sélection.

Un autre aspect porte sur une cellule hôte transformée par un vecteur d'expression ou un acide nucléique selon l'invention.

La cellule hôte peut être une cellule procaryote ou une cellule eucaryote. Des cellules hôtes couramment utilisées pour l'expression de cellules recombinantes incluent notamment des cellules de bactéries telles que *Escherichia coli,* des cellules de levures telles que *Saccharomyces cerevisiae,* des cellules de champignons tels que *Aspergillus niger,* des cellules d'insecte, et des cellules de mammifères (notamment humaines) telles que les lignées cellulaires CHO, HEK 293, PER-C6, *etc.*

La transformation des cellules procaryotes et des cellules eucaryotes est une technique bien connue de l'homme du métier. En fonction de la cellule à transformer, l'homme du métier pourra aisément déterminer les moyens nécessaires à l'introduction et à l'expression l'acide nucléique selon l'invention chez la cellule hôte choisie. Ainsi, le vecteur d'expression et la méthode d'introduction du vecteur d'expression au sein de la cellule hôte seront sélectionnés en fonction de la cellule hôte choisie.

La cellule hôte transformée par un vecteur d'expression ou un acide nucléique selon l'invention exprime préférentiellement le polypeptide selon l'invention de manière stable. L'homme du métier peut aisément vérifier que la cellule hôte exprime le polypeptide selon l'invention de manière stable, par exemple en utilisant la technique du Western Blot.

Les cellules hôtes selon l'invention sont notamment utiles pour produire des polypeptides selon l'invention. L'invention concerne donc une méthode de production d'un polypeptide selon l'invention comprenant l'étape de cultiver une cellule hôte selon l'invention dans des conditions permettant l'expression dudit polypeptide selon l'invention. Cette méthode peut en outre comprendre une étape de purification dudit polypeptide selon l'invention. Les étapes de culture et de purification peuvent par exemple être réalisées comme décrit dans l'exemple 2.

### Méthodes d'hydrolyse de polysaccharides selon l'invention

Les inventeurs ont trouvé que les enzymes PorA et PorB peuvent être utilisées pour hydrolyser du porphyrane, et permettent l'obtention d'oligosaccharides de structure et de taille définies. Plus particulièrement, l'hydrolyse du porphyrane par PorA et PorB permet d'obtenir des oligo-porphyranes, notamment du neo-porphyrobiose, du neoporphyrotétraose et/ou du neo-porphyrohexaose.

Un aspect de l'invention porte donc sur l'utilisation d'un polypeptide selon l'invention pour hydrolyser des polysaccharides et/ou pour produire des oligosaccharides.

Les polysaccharides utilisés dans le cadre de cette méthode sont des polysaccharides susceptibles de contenir des polysaccharides sulfatés, lesquels peuvent être ou non le composé majoritaire dudit polysaccharide. Préférentiellement, les polysaccharides utilisés dans le cadre de cette méthode comprennent ou consistent en du porphyrane. Alternativement, les polysaccharides utilisés dans le cadre de cette méthode peuvent comprendre ou consister en un polysaccharide différent du porphyrane mais constitué au moins en partie d'unités disaccharidiques constituées d'une unité D-galactose lié en β-1,4 avec une unité L-galactose modifiée par une O-sulfatation sur la position 06.

De tels polysaccharides sont présents chez les végétaux marins. Ils peuvent par exemple être obtenus à partir de végétaux marins, et en particulier à partir d'algues rouges, en utilisant des protocoles d'extraction bien connus de l'homme du métier, tels que la technique décrite dans Morrice et al. (Eur J Biochem. 1983 133:673-84). Ils peuvent éventuellement être séparés des autres composants éventuels par des méthodes de chromatographie liquide.

Dans un mode de réalisation préféré, le polypeptide selon l'invention est utilisé pour hydrolyser le porphyrane et/ou pour produire des oligo-porphyranes, notamment du neo-porphyrobiose, du neo-porphyrotétraose et/ou du neo-porphyrohexaose.

L'invention concerne également une méthode d'hydrolyse de polysaccharides et/ou de production d'oligosaccharides comprenant les étapes suivantes :
a) fournir un polypeptide selon l'invention ou une cellule hôte selon l'invention ; et
b) mettre ledit polypeptide ou ladite cellule hôte en contact avec un polysaccharide dans des conditions conduisant à l'obtention de polysaccharides hydrolysés, par exemple dans des conditions conduisant à une hydrolyse complète.

Comme cela apparaît clairement à la lecture de la présente demande, dans un mode de réalisation préféré, la méthode d'hydrolyse de polysaccharides et/ou de production d'oligosaccharides consiste en une méthode d'hydrolyse du porphyrane et/ou de production d'oligo-porphyranes, notamment de neo-porphyrobiose, de neo-porphyrotétraose et/ou de neo-porphyrohexaose. Les conditions conduisant à l'obtention de polysaccharides hydrolysés peuvent aisément être déterminées par l'homme du métier. Par exemple, les conditions décrites dans l'exemple 3 peuvent être utilisées. Le polypeptide ou la cellule hôte peut par exemple être mis en contact avec le polysaccharide à une température de 30°C. Lors de cette mise en contact, 6 µg de polypeptide peuvent par exemple être ajoutés à 50 ml d'une solution contenant 1 % (w/v) de polysaccharide. La durée d'incubation sera ajustée en fonction du polysaccharide hydrolysé que l'on souhaite obtenir. L'incubation peut par exemple durer environ ou au moins 6, 10 ou 14 heures. Ainsi, en partant d'une solution de polysaccharide contenant du porphyrane, il sera possible d'obtenir les oligosaccharides suivants en fonction de la durée d'incubation : neo-porphyrobiose, neoporphyrotétraose et/ou neo-porphyrohexaose.

La méthode selon l'invention peut éventuellement comprendre une étape (c) de purification desdits polysaccharides hydrolysés. Les techniques de purification de polysaccharides hydrolysés sont bien connues de l'homme du métier. La purification peut par exemple être réalisée par chromatographie d'exclusion, tel que décrit dans l'exemple 4. L'échantillon contenant les oligosaccharides peut par exemple être élué avec de l'ammonium carbonate 50mM à une vitesse de 1,5 mL/min pendant 650 minutes.

Dans un mode de réalisation préféré, les polysaccharides hydrolysés obtenus par la méthode selon l'invention ont un poids moléculaire moyen (*M_{w}*) inférieur ou égal à 5800, 5500, 5000, 4500, 4000, 3500 ou 3000 Da, encore préférentiellement inférieur ou égal à 5800 Da. Préférentiellement, les polysaccharides hydrolysés obtenus par la méthode selon l'invention possèdent un poids moléculaire inférieur 1270, 1170, 850, 750 ou 425 Da, par exemple environ 1261,04, 1163,97, 840,69, 743,62 ou 420,344 Da.

Dans un autre mode de réalisation préféré, les polysaccharides hydrolysés obtenus par la méthode selon l'invention comprennent au moins 25%, 50%, 60%, 70%, 80%, 90%, 95% ou 99% d'un ou plusieurs oligo-porphyranes choisis parmi le neo-porphyrobiose, le neo-porphyrotetraose et le neo-porphyrohexaose.

Par « neo-porphyrobiose », on entend l'unité disaccharide du porphyrane (laquelle est représentée sur la figure 1). Par « neo-porphyrotetraose », on entend un enchaînement de deux unités disaccharides du porphyrane. Par « neo-porphyrohexaose », on entend un enchaînement de trois unités disaccharides du porphyrane.

Les polysaccharides ainsi hydrolysés et purifiés peuvent ensuite être formulés en composition agroalimentaire, cosmétique ou pharmaceutique.

Enfin, les polysaccharides hydrolysés, en particulier, les oligo-porphyranes, notamment le neo-porphyrobiose, le en particulier, les oligo-porphyranes, notamment le neo-porphyrobiose, le neo-porphyrotétraose et/ou le neo-porphyrohexaose, susceptibles d'être obtenus par la méthode selon l'invention, sont décrits ainsi que des compositions agroalimentaires, cosmétiques et pharmaceutiques contenant de tels polysaccharides hydrolysés.

L'invention va maintenant être décrite en détail à l'aide de l'exposé expérimental ci-après, lequel illustre l'invention sans en limiter la portée.

### DESCRIPTION DES FIGURES

Figure 1 : Présentation schématique des unités disaccharidiques de l'agarose et du porphyrane.
Figure 2 : Activité enzymatique des porphyranases A et B. **A)** Activité enzymatique sur gel d'agarose. 1 µg des différents agarases et porphyranases est déposé sur une plaque de gel d'agarose de 1% et incubé pendant la nuit à 30°C. L'activité est ensuite révélée par coloration au lugol, indiqué par l'apparition d'un halo lumineux sur fond noir. A: ß-AgaraseA, B: ß-agaraseB, C: ß-agarases, D: ß-porphyranaseA, E: ß-porphyranaseB. **B)** Analyse cinétique de la digestion du porphyrane. Le porphyrane a été préalablement digéré par une β-agarase, et ne contient donc plus que des unités disaccharidiques du type porphyrane. Les lignes montrent la cinétique de digestion de PorA_CM (1), PorB (2), agaA (3) et agaB (4) de Z. *galactanivorans.*
Figure 3 : Analyse par HPLC des produits de dégradation du porphyrane obtenus avec l'enzyme PorA. DP2, DP4 et DP6 désignent les di-, tetra- et hexa-saccharides, qui ont été ensuite analysé par RMN.
Figure 4 : Spectre 1H-RMN des neo-porphyro-bi-, tetra-, et hexaoses, obtenus avec l'enzyme PorA.

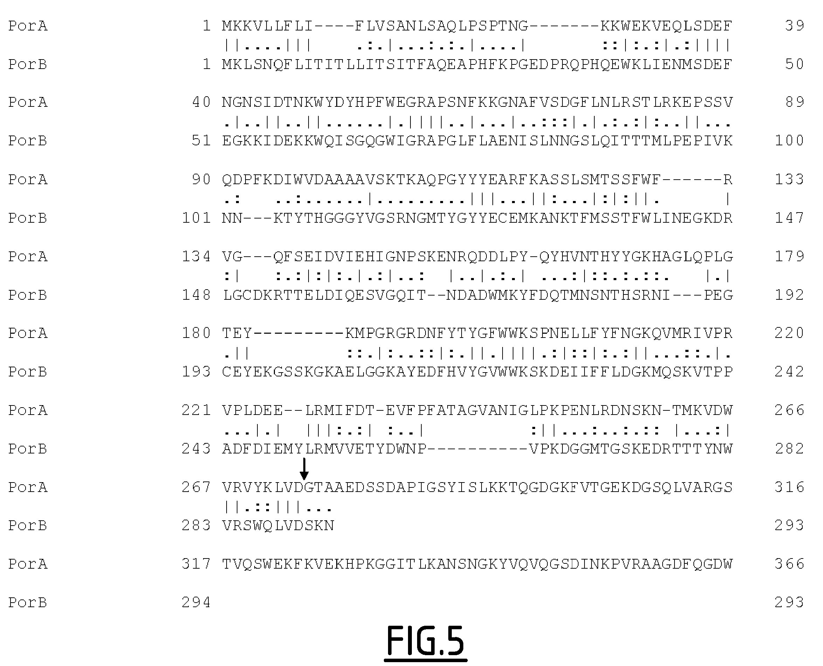

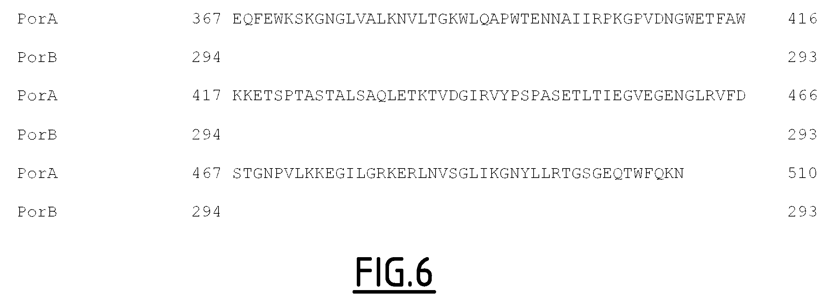

Figures 5 et 6 : Alignement de séquences entre PorA et PorB. La flèche indique l'extrémité C-terminale de PorA_CM.

### DESCRIPTION DES SEQUENCES DU LISTAGE DE SEQUENCES

SEQ ID NO : 1 représente la séquence nucléotidique de la porphyranase A.
SEQ ID NO : 2 représente la séquence protéique de la porphyranase A.
SEQ ID NO : 3 représente la séquence protéique de la porphyranase A recombinante produite dans l'exemple 2.
SEQ ID NO : 4 représente la séquence nucléotidique de la porphyranase B.
SEQ ID NO : 5 représente la séquence protéique de la porphyranase B.
SEQ ID NO : 6 représente la séquence protéique de la porphyranase B recombinante produite dans l'exemple 2.
SEQ ID Nos : 7 à 10 représentent des amorces convenant à cloner les gènes codant pour la porphyranase A et la porphyranase B.

### EXEMPLES

### Exemple 1 : Clonage des gènes de porphyranase A et B

Les cadres de lectures ouverts codant pour les enzymes PorA et PorB ont été construits sur la base des séquences du génome de *Z. galactanivorans.* Les gènes cibles ont été amplifié en parallèle par PCR, partant du matériel ADN génomique de *Z. galactanivorans,* avec des amorces 5' et 3' comme indiqué dans le Tableau 1 ci-dessous.

**Tableau 1 : Séquence des amorces pour le clonage de PorA_CM et PorB, calculée pour obtenir un Tm de ∼70 °C**

| Nom | SEQ ID NO : | Séquence |
|---|---|---|
| Amorce 5' | | |
| PorA_CM | 7 | ggggggggATCCCAATTACCATCTCCTACAAACGGG |
| PorB | 8 | ggggggggATCCCAAGAAGCTCCACATTTTAAGCCTG |
| Amorce 3' | | |
| PorA_CM | 9 | CCCCCCgAATTCTTAGTCAACCAATTTATACACCCGTACC |
| PorB | 10 | CCCCCCgAATTCTTAATTCTTTGAATCAACCAATTGCCATG |

Le gène entier de la ß-porphyranase A (contenant le domaine catalytique et les deux domaines ressemblant à des modules de fixation au substrat) a été tronqué pour produire une protéine recombinante contenant uniquement le domaine catalytique (résidus 19-275), qui sera appelé par la suite PorA_CM. PorB, dont la séquence indique un seul module appartenant dans son entier à la famille GH16, à été cloné intégralement pour obtenir la protéine recombinante active.

Les produits de l'amplification PCR ont ensuite été purifiés avec le kit Qiaquick PCR et élués avec 50 µl de H₂O. Les produits PCR purifiés ont à leur tour été digérés pendant trois heures à 37°C avec le mélange des enzymes de restriction BgIII/EcoRI dans leur tampons respectifs (NEB2 et BioLabs). Après digestion les produits ont été purifiés avec le kit Qiaquick PCR puis élués avec 25 µl de H₂O. Les produits de PCR ont été soumis à ligation avec le vecteur PFO4 (dérivé du vecteur pET15), préalablement déphosphorylé et digéré avec les enzymes de restrictions adéquates. Cette procédure de ligation a été effectuée avec la T4 ADN Ligase à 4°C durant une nuit.

Pour la procédure de transformation, des cellules de *E. coli* (souche DH5α), rendues compétentes par voie chimique, ont été utilisées. Pour cela, les cellules ont été mises sur un mélange glace, eau et NaCl pendant 30 minutes, puis ont été incubées pendant 30 minutes avec le mélange résultant de la ligation. La transformation a été effectuée en appliquant un choc thermique pendant 45 sec à 42°C, puis les cellules ont été remises dans le mélange glace, eau et NaCl pendant 30 minutes.

### Exemple 2 : Expression et purification des PorA CM et PorB:

PorA_CM et PorB ont été surexprimées dans des cellules de *E. coli* (souche BL21, DE3). La surexpression a été effectuée à 20 °C dans 1 L d'un milieu ZYP-5052 avec 100 µg ml⁻¹ d'ampicilline (Studier 2005 Protein Expr Purif. 41(1):207-34). Les cellules ont été récoltées par centrifugation (4000g, 20 min, 4°C) après 3 jours et demi de culture, (OD finale à 600 nm: environ 16). Le culot a ensuite été suspendu dans du tampon A (20 mM Tris pH 8, 200 mM NaCl, 20 mM Imidazole, pH 7.5, Lysozyme, DNAse). Les cellules ainsi suspendues ont été lysées par du lysozyme pendant 30 minutes sur glace, puis ont fait l'objet d'une sonication. Le lysat a été clarifié par centrifugation (50 000g, 30 min, 4°C), puis par une filtration en utilisant des filtres (Millipore) de 0.2 µm. La solution ainsi filtrée a été chargée sur une colonne de 10 ml de résine IMAC HyperCell (Pall Corporation), laquelle a été chargé avec une solution NiSO₄. La colonne avait préalablement été équilibrée avec le tampon A, dénué de lysozyme et de DNAse. Après une étape de lavage avec le tampon A (dix volumes de colonne), la protéine a été éluée, à 1 ml min⁻¹, dans 60 ml en effectuant un gradient linéaire entre le tampon A et un tampon A additionné de 60 % de tampon B (20 mM Tris pH 8, 200 mM NaCl et 500 mM Imidazole). Les protéines ont été concentrées par ultrafiltration sur une membrane Amicon (polyethersulfone, taille limite 30 kDa), afin d'obtenir un volume finale d'environ 5ml. Une deuxième étape de purification a ensuite été effectuée sur une colonne Sephacryl S-200 (GE Healthcare) pré-équilibrée avec un tampon C (20 mM Tris pH 8, 4 % (v/v) Glycerol pour PorA_CM, et 20 mM Tris pour PorB) à une vitesse de 1 ml min⁻¹. Toutes les fractions contenant la protéine pur (comme analysé par SDS-PAGE) ont été additionnées et concentrées par filtration/centrifugation (Amicon, taille limite 10 kDa) à 2.6 mg/ml pour PorA_CM et environ 8 mg/ml pour PorB. Toutes procédures de chromatographie ont été effectuées avec un système ÄKTA Explorer (GE-Healthcare) à température ambiante.

### Exemple 3 : Dégradation enzymatique du porphyrane

Une solution contenant 1 % (w/v) du polysaccharide a été utilisé pour l'hydrolyse enzymatique avec les porphyranases A et B. Un total de 50 ml de la solution polysaccharidique a été incubé avec 6 µg d'enzyme à 30°C pendant une nuit. Des aliquotes ont été prélevés tout au long de l'hydrolyse jusqu'à ce que l'hydrolyse complète soit atteinte (environ 14 heures).

Après digestion complète par l'enzyme, la solution a été chauffée à 96° C pendant 30 min pour inactiver une éventuelle activité résiduelle de l'enzyme. La solution a ensuite été centrifugée pendant 20 min à 5000g et 4°C et le surnageant a été filtré avec des filtres seringues Millipore de 0.2 µm. Le polysaccharide digéré a été congelé à -80° C puis lyophilisé.

Les aliquotes ont été analysés par la méthode de détection des sucres réducteurs en mélangeant 10 µl d'aliquote de protéine avec 90 µl d'eau (dilution 10x), puis ensuite avec 1ml de la solution ferricyanide (300 mg potassium hexocyanoferrate III, 29 g Na2CO3, 1 mL 5 M NaOH, complété à 1 L avec de l'eau). Le mélange a été chauffé à 100°C pendant 15 min, puis refroidi à température ambiante pour lire l'absorbance à 420 nm.

### Exemple 4: Purification des oligo-porphyranes par chromatographie d'exclusion préparative

La purification des oligo-porphyranes a été effectuée par chromatographie d'exclusion préparative avec trois colonnes Superdex 30 (26/60) de GE HealthCare en série, intégrées sur un système HPLC system injecteur/collecteur liquide (Gilson). Les oligosaccharides ont été détectés par leur index réfractif (détecteur Spectra System RI-50) et l'intégration des données (Gilson et détecteur réfraction) par le logiciel Unipoint (Gilson).

Le produit de digestion polysaccharidique, congelé et lyophilisé, a été dissous dans de l'eau déminéralisée pour obtenir une solution de 4% (w/v). Après filtration sur une membrane poreuse de 0.45µm, 4 mL de l'échantillon a été injecté, puis élué avec de l'ammonium Carbonate [(NH₄)₂CO₃] 50mM à une vitesse de 1.5 mL/min pendant 650 minutes. Les fractions d'oligosaccharides ont été collectées et directement analysées par HPLC.

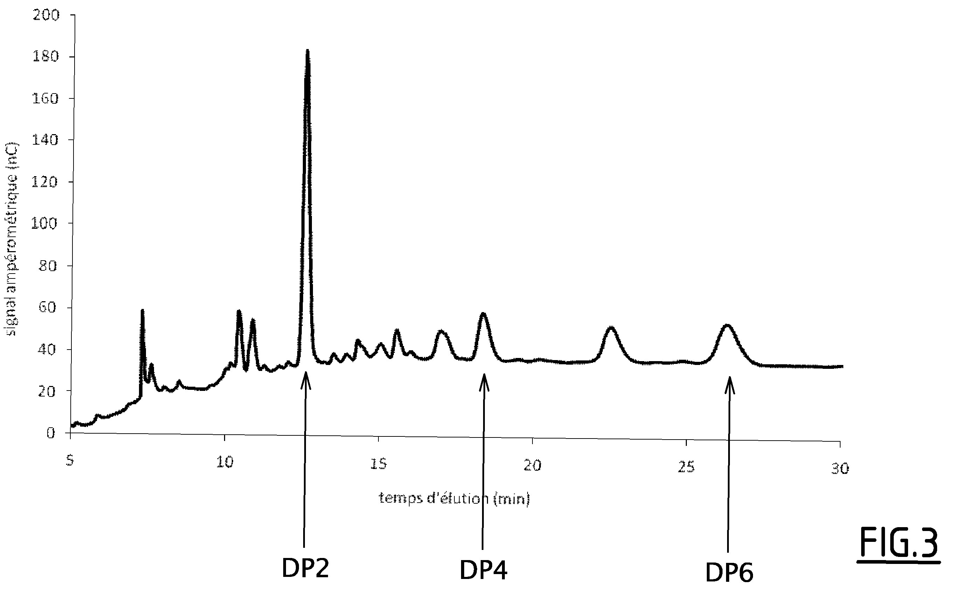

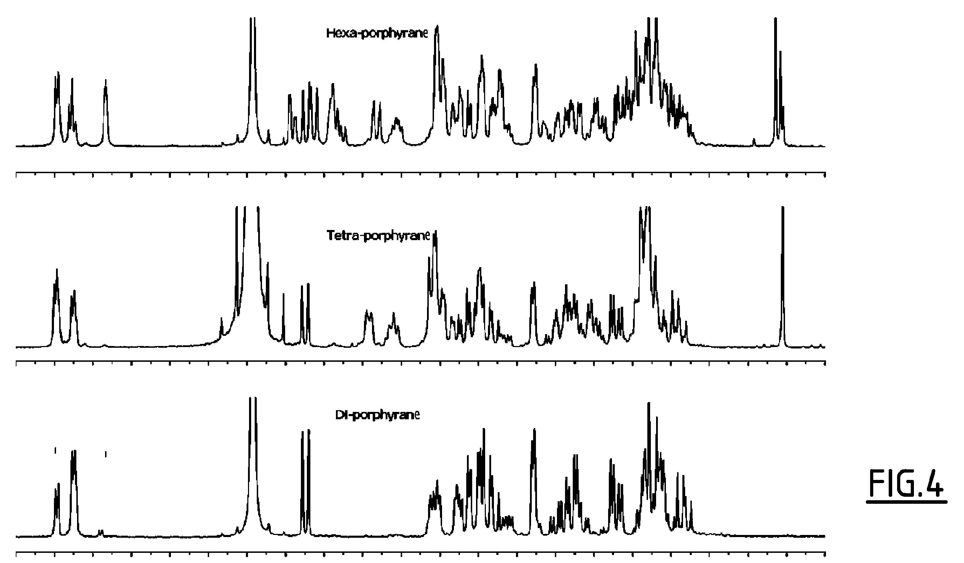

Selon ce procédé, il est possible de préparer des oligosaccharides composés uniquement du motif de répétition porphyrane ou des oligosaccharides hybrides contenant des motifs porphyrane associés à des motifs de type agarose. Ainsi nous avons isolé et purifié des oligo-porphyranes du disaccharide au hexasaccharide (Figures 3 et 4), correspondant au poids moléculaires de 420.344 Da (neo-porphyrobiose, appelé di-porphyrane sur la Figure 4), 840.69 et/ou 743.62 Da (neo-porphyrotetraose, appelé tetra-porphyrane sur la Figure 4) et 1261.04 et/ou 1163.97 Da (neo-porphyrohexaose, appelé hexa-porphyrane sur la Figure 4), respectivement.

### Exemple 5 : Résultats et discussion

Le séquençage du génome complet de la bactérie *Zobellia galactanivorans* (déposée à la Collection DSMZ le 8 mai 1998 sous le numéro DSM 12170) a été réalisé. Le séquençage a permis d'identifier seize gènes appartenant à la famille GH16 des glycoside hydrolases. Parmi ces seize gènes, les gènes codant pour la κ-carraghénase CgkA et les β-agarases AgaA et AgaB sont déjà connus et caractérisés (Barbeyron et al. Mol Biol Evol. 1998 15:528-37 ; Jam et al. Biochem J. 2005 385 :703-13 ; Allouch et al. J Biol Chem. 2003 278:47171-80).

Deux autres gènes appartenant à la famille GH16 des glycoside hydrolases ont été clonés comme exposé dans l'exemple 1 ci-dessus. Ces gènes, dénommés *porA* et *porB,* codent pour des protéines dénommées PorA et PorB respectivement. La protéine PorA est composée de 510 acides aminés et a une masse moléculaire théorique de 57,311 kDa. Après élimination du peptide signal, cette protéine a une masse moléculaire calculée de 55,193 kDa. La protéine PorB est composée de 293 acides aminés. Après élimination du peptide signal, cette protéine a une masse moléculaire théorique de 31,271 kDa.

Comme montré dans le tableau 2 ci-dessous, ces deux gènes présentent moins de 25% d'identité avec les β-agarases et la κ-carrghénase de *Zobellia galactanivorans.*

**Tableau 2 : Matrice d'identité de séquence entre des modules catalytiques de certaines glycoside hydrolases de la famille GH16 de Zobellia galactanivorans.**

| | 1 | 2 | 3 | 4 | 5 |
|---|---|---|---|---|---|
| 1. AgaA | | 35.6 | 24.2 | 25.2 | 22.8 |
| 2. AgaB | | | 18.4 | 22.0 | 20.0 |
| 3. CgkA | | | | 23.3 | 21.0 |
| 4. PorA | | | | | 26.9 |
| 5. PorB | | | | | |

Une fois le clonage effectué, les protéines PorA et PorB ont été surexprimées chez *Escherichia coli* comme exposé dans l'exemple 2.

L'activité des protéines PorA et PorB sur plusieurs galactanes d'algues rouges a ensuite été étudiée (exemple 3). Il a été constaté que ces enzymes n'ont aucune activité sur les carraghénanes ou sur l'agarose (Figure 2A). En revanche, ces enzymes présentent une forte activité sur le porphyrane (Figure 2B).

La purification des oligosaccharides produits et leur caractérisation par ¹H-RMN (exemple 4) démontre que les enzymes PorA et PorB hydrolysent spécifiquement le porphyrane et clivent les liaisons β-1,4 entre les unités D-galactose et les unités L-galactose sulfaté en position 6. La sulfatation est nécessaire pour l'activité de l'enzyme et est exclusive par rapport aux activités β-agarases, comme le montre l'activité de PorA et PorB sur du porphyrane, prédigéré par une β-agarase (Figure 2B).

Comme montré dans l'exemple 4, l'hydrolyse du porphyrane par PorA et PorB permet d'obtenir des mélanges homogènes de neoporphyrobiose, neo-porphyrotétraose et/ou neo-porphyrohexaose.

Par conséquent, les protéines PorA et PorB sont les premières β-porphyranases décrites à ce jour.

## Revendications

1. Polypeptide isolé **caractérisé en ce qu'**il possède une activité porphyranase, comprenant une séquence choisie parmi :
(i) une séquence au moins 90 % identique aux résidus 19 à 274 de la séquence SEQ ID NO : 2,
(ii) une séquence au moins 90 % identique aux résidus 22 à 293 de la séquence SEQ ID NO : 5, et
(iii) un fragment d'au moins 200 aminés consécutifs de la séquence (i) ou (ii).

2. Polypeptide selon la revendication 1, où ledit polypeptide est un polypeptide recombinant.

3. Polypeptide selon l'une quelconque des revendications précédentes, comprenant une séquence choisie parmi :
a) une séquence au moins 95% identique aux résidus :
- 19 à 274 de la séquence SEQ ID NO : 2 ; ou
- 22 à 293 de la séquence SEQ ID NO : 5;
b) un fragment d'au moins 250 acides aminés consécutifs de la séquence SEQ ID NO : 2 ou SEQ ID NO : 5.

4. Acide nucléique isolé codant pour un polypeptide tel que défini à l'une quelconque des revendications 1 à 3.

5. Vecteur d'expression comprenant un acide nucléique selon la revendication 4.

6. Cellule hôte transformée par un vecteur d'expression selon la revendication 5.

7. Utilisation d'un polypeptide selon l'une quelconque des revendications 1 à 3 pour hydrolyser des polysaccharides.

8. Méthode d'hydrolyse de polysaccharides comprenant les étapes suivantes:
a) fournir un polypeptide tel que défini à l'une quelconque des revendications 1 à 3, ou une cellule hôte telle que définie à la revendication 6 ; et
b) mettre ledit polypeptide ou ladite cellule hôte en contact avec un polysaccharide dans des conditions permettant l'obtention de polysaccharides hydrolysés.

9. Méthode selon la revendication 8, comprenant en outre une étape (c) de purification desdits polysaccharides hydrolysés.

10. Méthode selon la revendication 9, comprenant en outre une étape (d) de formulation desdits polysaccharides hydrolysés et purifiés en composition agroalimentaire, cosmétique ou pharmaceutique.

11. Méthode selon l'une quelconque des revendications 8 à 10, où lesdits polysaccharides contiennent du porphyrane.

## Patentansprüche

1. Isoliertes Polypeptid, **dadurch gekennzeichnet, dass** es eine Porphyranase-Aktivität besitzt, mit einer Sequenz, die ausgewählt ist aus:
(i) einer Sequenz, die zu mindestens 90 % mit den Resten 19 bis 274 der Sequenz SEQ ID NR.: 2 identisch ist,
(ii) einer Sequenz, die zu mindestens 90 % mit den Resten 22 bis 293 der Sequenz SEQ ID NR.: 5 identisch ist, und
(iii) einem Fragment von mindestens 200 aufeinander folgenden Aminosäuren der Sequenz (i) oder (ii).

2. Polypeptid nach Anspruch 1, wobei das Polypeptid ein rekombinantes Polypeptid ist.

3. Polypeptid nach einem der vorangehenden Ansprüche mit einer Sequenz, die ausgewählt ist aus:
a) einer Sequenz, die zu mindestens 95 % identisch ist mit den Resten:
- 19 bis 274 der Sequenz SEQ ID NR.: 2; oder
- 22 bis 293 der Sequenz SEQ ID NR.: 5;
b) einem Fragment von mindestens 250 aufeinander folgenden Aminosäuren der Sequenz SEQ ID NR.: 2 oder SEQ ID NR.: 5.

4. Isolierte Nukleinsäure, die ein Polypeptid, wie in einem der Ansprüche 1 bis 3 definiert, codiert.

5. Expressionsvektor mit einer Nukleinsäure nach Anspruch 4.

6. Durch einen Expressionsvektor nach Anspruch 5 transformierte Wirtszelle.

7. Verwendung eines Polypeptids nach einem der Ansprüche 1 bis 3 zum Hydrolysieren von Polysacchariden.

8. Verfahren zur Hydrolyse von Polysacchariden mit den folgenden Schritten:
a) Liefern eines Polypeptids, wie in einem der Ansprüche 1 bis 3 definiert, oder einer Wirtszelle, wie in Anspruch 6 definiert; und
b) Bringen des Polypeptids oder der Wirtszelle in Kontakt mit einem Polysaccharid unter Bedingungen, die das Erhalten von hydrolysierten Polysacchariden ermöglichen.

9. Verfahren nach Anspruch 8, das außerdem einen Schritt (c) zur Reinigung der hydrolysierten Polysaccharide umfasst.

10. Verfahren nach Anspruch 9 das außerdem einen Schritt (d) zur Formulierung der hydrolysierten und gereinigten Polysaccharide in einer Nahrungsmittel-, kosmetischen oder pharmazeutischen Zusammensetzung umfasst.

11. Verfahren nach einem der Ansprüche 8 bis 10, wobei die Polysaccharide Porphyran enthalten.

## Claims

1. An isolated polypeptide, **characterized in that** it has porphyranase activity, comprising a sequence selected from :
i) a sequence at least 90% identical to the residues 19 to 274 of the sequence SEQ ID NO: 2; or
ii) a sequence at least 90% identical to the residues 22 to 293 of the sequence SEQ ID NO: 5;
iii) a fragment of at least 200 consecutive amino acids of the sequence i) or ii).

2. The polypeptide according to claim 1, wherein said polypeptide is a recombinant polypeptide.

3. The polypeptide according to any of the preceding claims, comprising a sequence selected from :
a) a sequence at least 95% identical to the residues:
- 19 to 274 of the sequence SEQ ID NO: 2; or
- 22 to 293 of the sequence SEQ ID NO: 5;
a) b) a fragment of at least 250 consecutive amino acids of the sequence SEQ ID NO: 2 or SEQ ID NO: 5.

4. An isolated nucleic acid coding for a polypeptide as defined according to any of claims 1 to 3.

5. An expression vector comprising a nucleic acid according to claim 4.

6. A host cell transformed by an expression vector according to claim 5.

7. The use of a polypeptide according to any of claims 1 to 3 for hydrolyzing polysaccharides.

8. A method for hydrolyzing polysaccharides comprising the following steps:
a) providing a polypeptide as defined according to any of claims 1 to 3, or a host cell as defined in claim 6; and
b) putting said polypeptide or said host cell in contact with a polysaccharide under conditions allowing hydrolyzed polysaccharides to be obtained.

9. The method according to claim 8, further comprising a step (c) for purifying said hydrolyzed polysaccharides.

10. The method according to claim 9, further comprising a step (d) for formulating said hydrolyzed and purified polysaccharides in an agrifood, cosmetic or pharmaceutical composition.

11. The method according to any of claims 8 to 10, wherein said polysaccharides contain porphyran.


### SEQUENCE LISTING

<110> CENTRE NATIONAL DE LA RECHERCHE SCIENTIFIQUE (C.N.R.S)
<120> Porphyranases et leur utilisation pour hydrolyser des polysaccharides
<130> BET10P1064
<160> 10
<170> PatentIn version 3.4
<210> 1
   <211> 1533
   <212> DNA
   <213> Zobellia galactanivorans
<220>
   <221> sig_peptide
   <222> (1)..(54)
<400> 1
   atgaaaaaag tattactatt tttaattttt ttagtaagcg caaatctttc tgcgcaatta      60
   ccatctccta caaacgggaa gaaatgggaa aaggttgaac aattatccga tgaatttaac     120
   ggcaactcca ttgacaccaa taagtggtat gattaccacc ctttttggga gggccgagcc     180
   cccagcaatt ttaaaaaagg gaatgctttc gtaagtgacg gtttcctaaa ccttcgttct     240
   acattaagaa aagaaccaag tagcgttcaa gatcctttta aggatatttg ggtagatgct     300
   gccgcggcgg tttccaagac caaggcccaa cccggatatt attacgaggc tcgctttaag     360
   gcctcctctt tgtctatgac ttcttcgttt tggttccgcg ttggccaatt ttctgaaatc     420
   gatgttatcg agcatattgg aaatccatcc aaagaaaacc gacaggacga tcttccttat     480
   caatatcatg ttaatacaca ttattacgga aaacatgccg gattgcaacc acttggaacc     540
   gaatataaaa tgcccggcag gggaagggat aatttttaca cctatggatt ctggtggaag     600
   agtcctaatg aacttctttt ttacttcaat ggcaagcagg ttatgcgtat cgttccaaga     660
   gtgcctttag acgaggaatt aagaatgatt tttgacacag aagtattccc ttttgccacg     720
   gcaggtgttg ccaatatcgg acttcctaag ccggaaaacc tccgtgacaa ttctaaaaac     780
   accatgaaag tagattgggt acgggtgtat aaattggttg acggcaccgc cgcggaagac     840
   agttccgatg ctccgatcgg cagttacatt tccctcaaaa aaacgcaagg cgacggtaaa     900
   tttgtaaccg gtgaaaaaga tggcagccaa ttggttgcaa gaggctcgac cgttcaaagc     960
   tgggaaaaat tcaaagtaga aaaacaccct aaaggcggga ttacccttaa ggccaattcc    1020
   aatggtaaat atgtacaagt tcaaggaagc gacatcaaca aaccggtaag ggccgcgggc    1080
   gattttcagg gcgattggga acaattcgaa tggaaatcca aaggaaacgg acttgtagcc    1140
   ttgaaaaacg tgcttacggg caaatggcta caggctccat ggaccgaaaa caacgcgata    1200
   atccgaccga aagggcccgt agataatggt tgggaaactt ttgcttggaa aaaggagaca    1260
   tcacctaccg ccagtacggc gctttccgcc cagttggaga caaagaccgt agatggaata   1320
   agagtgtatc ccagccccgc atcggaaacc ttgactattg agggagtgga gggcgaaaat   1380
   ggtttacgtg tttttgactc gacgggcaat ccagtcctta aaaaagaagg catactaggc   1440
   cgtaaagaac gattaaacgt ttccggtctt atcaaaggca actacctatt gcgcactgga   1500
   tcaggtgaac aaacctggtt tcagaaaaac tga                                1533
<210> 2
   <211> 510
   <212> PRT
   <213> Zobellia galactanivorans
<220>
   <221> SIGNAL
   <222> (1)..(18)
<400> 2
   Met Lys Lys Val Leu Leu Phe Leu Ile Phe Leu Val Ser Ala Asn Leu
   1               5                   10                  15
   Ser Ala Gln Leu Pro Ser Pro Thr Asn Gly Lys Lys Trp Glu Lys Val
               20                  25                  30
   Glu Gln Leu Ser Asp Glu Phe Asn Gly Asn Ser Ile Asp Thr Asn Lys
           35                  40                  45
   Trp Tyr Asp Tyr His Pro Phe Trp Glu Gly Arg Ala Pro Ser Asn Phe
       50                  55                  60
   Lys Lys Gly Asn Ala Phe Val Ser Asp Gly Phe Leu Asn Leu Arg Ser
   65                  70                  75                  80
   Thr Leu Arg Lys Glu Pro Ser Ser Val Gln Asp Pro Phe Lys Asp Ile
                   85                  90                  95
   Trp Val Asp Ala Ala Ala Ala Val Ser Lys Thr Lys Ala Gln Pro Gly
               100                 105                 110
   Tyr Tyr Tyr Glu Ala Arg Phe Lys Ala Ser Ser Leu Ser Met Thr Ser
           115                 120                 125
   Ser Phe Trp Phe Arg Val Gly Gln Phe Ser Glu Ile Asp Val Ile Glu
       130                 135                 140
   His Ile Gly Asn Pro Ser Lys Glu Asn Arg Gln Asp Asp Leu Pro Tyr
   145                 150                 155                 160
   Gln Tyr His Val Asn Thr His Tyr Tyr Gly Lys His Ala Gly Leu Gln
                   165                 170                 175
   Pro Leu Gly Thr Glu Tyr Lys Met Pro Gly Arg Gly Arg Asp Asn Phe
               180                 185                 190
   Tyr Thr Tyr Gly Phe Trp Trp Lys Ser Pro Asn Glu Leu Leu Phe Tyr
           195                 200                 205
   Phe Asn Gly Lys Gln Val Met Arg Ile Val Pro Arg Val Pro Leu Asp
       210                 215                 220
   Glu Glu Leu Arg Met Ile Phe Asp Thr Glu Val Phe Pro Phe Ala Thr
   225                 230                 235                 240
   Ala Gly Val Ala Asn Ile Gly Leu Pro Lys Pro Glu Asn Leu Arg Asp
                   245                 250                 255
   Asn Ser Lys Asn Thr Met Lys Val Asp Trp Val Arg Val Tyr Lys Leu
               260                 265                 270
   Val Asp Gly Thr Ala Ala Glu Asp Ser Ser Asp Ala Pro Ile Gly Ser
           275                 280                 285
   Tyr Ile Ser Leu Lys Lys Thr Gln Gly Asp Gly Lys Phe Val Thr Gly
       290                 295                 300
   Glu Lys Asp Gly Ser Gln Leu Val Ala Arg Gly Ser Thr Val Gln Ser
   305                 310                 315                 320
   Trp Glu Lys Phe Lys Val Glu Lys His Pro Lys Gly Gly Ile Thr Leu
                   325                 330                 335
   Lys Ala Asn Ser Asn Gly Lys Tyr Val Gln Val Gln Gly Ser Asp Ile
               340                 345                 350
   Asn Lys Pro Val Arg Ala Ala Gly Asp Phe Gln Gly Asp Trp Glu Gln
           355                 360                 365
   Phe Glu Trp Lys Ser Lys Gly Asn Gly Leu Val Ala Leu Lys Asn Val
       370                 375                 380
   Leu Thr Gly Lys Trp Leu Gln Ala Pro Trp Thr Glu Asn Asn Ala Ile
   385                 390                 395                 400
   Ile Arg Pro Lys Gly Pro Val Asp Asn Gly Trp Glu Thr Phe Ala Trp
                   405                 410                 415
   Lys Lys Glu Thr Ser Pro Thr Ala Ser Thr Ala Leu Ser Ala Gln Leu
               420                 425                 430
   Glu Thr Lys Thr Val Asp Gly Ile Arg Val Tyr Pro Ser Pro Ala Ser
           435                 440                 445
   Glu Thr Leu Thr Ile Glu Gly Val Glu Gly Glu Asn Gly Leu Arg Val
       450                 455                 460
   Phe Asp Ser Thr Gly Asn Pro Val Leu Lys Lys Glu Gly Ile Leu Gly
   465                 470                 475                 480
   Arg Lys Glu Arg Leu Asn Val Ser Gly Leu Ile Lys Gly Asn Tyr Leu
                   485                 490                 495
   Leu Arg Thr Gly Ser Gly Glu Gln Thr Trp Phe Gln Lys Asn
               500                 505                 510
<210> 3
   <211> 264
   <212> PRT
   <213> Artificial
<220>
   <223> Porphyranase A recombinante
<220>
   <221> MISC_FEATURE
   <222> (1)..(8)
   <223> Introduit par le vecteur
<220>
   <221> MISC_FEATURE
   <222> (9)..(64)
   <223> Porphyranase
<400> 3
   His His His His His His Gly Ser Gln Leu Pro Ser Pro Thr Asn Gly
   1               5                   10                  15
   Lys Lys Trp Glu Lys Val Glu Gln Leu Ser Asp Glu Phe Asn Gly Asn
               20                  25                  30
   Ser Ile Asp Thr Asn Lys Trp Tyr Asp Tyr His Pro Phe Trp Glu Gly
           35                  40                  45
   Arg Ala Pro Ser Asn Phe Lys Lys Gly Asn Ala Phe Val Ser Asp Gly
       50                  55                  60
   Phe Leu Asn Leu Arg Ser Thr Leu Arg Lys Glu Pro Ser Ser Val Gln
   65                  70                  75                  80
   Asp Pro Phe Lys Asp Ile Trp Val Asp Ala Ala Ala Ala Val Ser Lys
                   85                  90                  95
   Thr Lys Ala Gln Pro Gly Tyr Tyr Tyr Glu Ala Arg Phe Lys Ala Ser
               100                 105                 110
   Ser Leu Ser Met Thr Ser Ser Phe Trp Phe Arg Val Gly Gln Phe Ser
           115                 120                 125
   Glu Ile Asp Val Ile Glu His Ile Gly Asn Pro Ser Lys Glu Asn Arg
       130                 135                 140
   Gln Asp Asp Leu Pro Tyr Gln Tyr His Val Asn Thr His Tyr Tyr Gly
   145                 150                 155                 160
   Lys His Ala Gly Leu Gln Pro Leu Gly Thr Glu Tyr Lys Met Pro Gly
                   165                 170                 175
   Arg Gly Arg Asp Asn Phe Tyr Thr Tyr Gly Phe Trp Trp Lys Ser Pro
               180                 185                 190
   Asn Glu Leu Leu Phe Tyr Phe Asn Gly Lys Gln Val Met Arg Ile Val
           195                 200                 205
   Pro Arg Val Pro Leu Asp Glu Glu Leu Arg Met Ile Phe Asp Thr Glu
       210                 215                 220
   Val Phe Pro Phe Ala Thr Ala Gly Val Ala Asn Ile Gly Leu Pro Lys
   225                 230                 235                 240
   Pro Glu Asn Leu Arg Asp Asn Ser Lys Asn Thr Met Lys Val Asp Trp
                   245                 250                 255
   Val Arg Val Tyr Lys Leu Val Asp
               260
<210> 4
   <211> 882
   <212> DNA
   <213> Zobellia galactanivorans
<220>
   <221> six_peptide
   <222> (1)..(63)
<400> 4
   atgaagcttt ccaaccaatt cctaataaca attaccctac taatcacaag tattacattc      60
   gctcaagaag ctccacattt taagcctgga gaagacccaa ggcaacctca tcaggaatgg     120
   aaattaattg aaaacatgtc cgatgaattt gaagggaaaa agatagatga aaaaaaatgg     180
   cagatttcgg gccaaggatg gataggtcgc gctccgggat tgtttcttgc agaaaatatt     240
   tccttaaata acggaagttt acaaataact acaactatgc tgccagaacc tatagtcaaa     300
   aacaataaaa cctatacgca cggaggtggt tacgttggct caagaaatgg tatgacctat     360
   ggctattatg agtgcgaaat gaaagccaac aaaaccttca tgtcctctac attttggtta     420
   attaatgaag ggaaagaccg attaggatgc gacaaaagaa caacggaatt ggacattcag     480
   gaatctgttg gacaaataac gaatgatgcc gactggatga aatactttga ccaaaccatg     540
   aactccaata cccacagtag aaatattccg gagggatgtg aatacgaaaa aggatcgagc     600
   aaaggcaaag cggagttagg aggaaaagca tacgaagatt tccatgttta tggtgtttgg     660
   tggaaatcta aggatgaaat tatatttttc ttggacggca agatgcaatc taaagtaacg     720
   cccccggccg attttgatat tgaaatgtat ttacgaatgg tcgttgagac ctatgattgg     780
   aatccggtac ctaaagacgg cggcatgaca ggttcaaaag aagatagaac tacaacgtac     840
   aattgggtaa ggtcatggca attggttgat tcaaagaatt aa                        882
<210> 5
   <211> 293
   <212> PRT
   <213> Zobellia galactanivorans
<220>
   <221> SIGNAL
   <222> (1)..(21)
<400> 5
   Met Lys Leu Ser Asn Gln Phe Leu Ile Thr Ile Thr Leu Leu Ile Thr
   1               5                   10                  15
   Ser Ile Thr Phe Ala Gln Glu Ala Pro His Phe Lys Pro Gly Glu Asp
               20                  25                  30
   Pro Arg Gln Pro His Gln Glu Trp Lys Leu Ile Glu Asn Met Ser Asp
           35                  40                  45
   Glu Phe Glu Gly Lys Lys Ile Asp Glu Lys Lys Trp Gln Ile Ser Gly
       50                  55                  60
   Gln Gly Trp Ile Gly Arg Ala Pro Gly Leu Phe Leu Ala Glu Asn Ile
   65                  70                  75                  80
   Ser Leu Asn Asn Gly Ser Leu Gln Ile Thr Thr Thr Met Leu Pro Glu
                   85                  90                  95
   Pro Ile Val Lys Asn Asn Lys Thr Tyr Thr His Gly Gly Gly Tyr Val
               100                 105                 110
   Gly Ser Arg Asn Gly Met Thr Tyr Gly Tyr Tyr Glu Cys Glu Met Lys
           115                 120                 125
   Ala Asn Lys Thr Phe Met Ser Ser Thr Phe Trp Leu Ile Asn Glu Gly
       130                 135                 140
   Lys Asp Arg Leu Gly Cys Asp Lys Arg Thr Thr Glu Leu Asp Ile Gln
   145                 150                 155                 160
   Glu Ser Val Gly Gln Ile Thr Asn Asp Ala Asp Trp Met Lys Tyr Phe
                   165                 170                 175
   Asp Gln Thr Met Asn Ser Asn Thr His Ser Arg Asn Ile Pro Glu Gly
               180                 185                 190
   Cys Glu Tyr Glu Lys Gly Ser Ser Lys Gly Lys Ala Glu Leu Gly Gly
           195                 200                 205
   Lys Ala Tyr Glu Asp Phe His Val Tyr Gly Val Trp Trp Lys Ser Lys
       210                 215                 220
   Asp Glu Ile Ile Phe Phe Leu Asp Gly Lys Met Gln Ser Lys Val Thr
   225                 230                 235                 240
   Pro Pro Ala Asp Phe Asp Ile Glu Met Tyr Leu Arg Met Val Val Glu
                   245                 250                 255
   Thr Tyr Asp Trp Asn Pro Val Pro Lys Asp Gly Gly Met Thr Gly Ser
               260                 265                 270
   Lys Glu Asp Arg Thr Thr Thr Tyr Asn Trp Val Arg Ser Trp Gln Leu
           275                 280                 285
   Val Asp Ser Lys Asn
       290
<210> 6
   <211> 280
   <212> PRT
   <213> Artificial
<220>
   <223> Porphyranase B recombinante
<220>
   <221> MISC_FEATURE
   <222> (1)..(8)
   <223> Introduit par le vecteur
<220>
   <221> MISC_FEATURE
   <222> (9) .. (280)
   <223> Porphyranase
<400> 6 Trp Gln Leu Val Asp Ser Lys Asn 275 280
<210> 7
   <211> 36
   <212> DNA
   <213> Artificial
<220>
   <223> amorce
<400> 7
   ggggggggat cccaattacc atctcctaca aacggg 36
<210> 8
   <211> 37
   <212> DNA
   <213> Artificial
<220>
   <223> amorce
<400> 8
   ggggggggat cccaagaagc tccacatttt aagcctg 37
<210> 9
   <211> 40
   <212> DNA
   <213> Artificial
<220>
   <223> amorce
<400> 9
   ccccccgaat tcttagtcaa ccaatttata cacccgtacc 40
<210> 10
   <211> 41
   <212> DNA
   <213> Artificial
<220>
   <223> amorce
<400> 10
   ccccccgaat tcttaattct ttgaatcaac caattgccat g 41